# EUROPEAN PATENT SPECIFICATION

(11) **EP 3 697 881 B1**
(45) Date of publication and mention of the grant of the patent: **18.12.2024**
(21) Application number: 18789584.2
(22) Date of filing: 15.10.2018
(51) Int. Cl.: C11D 3/386, C11D 3/38, C11D 3/43, C11D 3/20, C11D 7/50, C12N 9/98

(54) **LOW DUSTING GRANULES**
STAUBARME GRANULATE
GRANULÉS DE POUDRAGE FAIBLE

(30) Priority: 16.10.2017 EP 17196659
(43) Date of publication of application: 26.08.2020
(73) Proprietor: Novozymes A/S, 2880 Bagsvaerd (DK)
(72) Inventor: SIMONSEN, Ole, 2880 Bagsvaerd (DK); BACH, Poul, 2880 Bagsvaerd (DK); CERVERA-PADRELL, Albert E., 2880 Bagsvaerd (DK); JENSEN, Lars, 2880 Bagsvaerd (DK); SHANG, Lei, 2880 Bagsvaerd (DK); NISSEN, Lotte Elisabeth, 2880 Bagsvaerd (DK)
(74) Representative: NZ EPO Representatives
(86) International application number: PCT/EP2018/078115
(87) International publication number: WO 2019/076833

(56) References cited:
- EP-A2- 0 304 332
- EP-B1- 0 304 332
- WO-A1-2013/007594
- WO-A1-2017/157781
- JP-A- 2003 000 234
- US-A- 4 106 991
- US-A- 4 242 219
- US-A1- 2003 054 511
- US-A1- 2007 111 920

## Description

### FIELD OF THE INVENTION

The present invention relates to a method for preparing a plurality of granules comprising 6-30% w/w of a non-volatile liquid by using mixer-granulation. The granules exhibit reduced release of active dust, such as enzyme dust, after subjecting the granules to mechanical stress.

### BACKGROUND

One of the major challenges for the development of enzyme formulations has been avoiding enzyme dust exposure, which is known to cause irritation or allergenic reactions. Since the 1970s, several breakthroughs were made in this field, to the point that potential dust exposure only occurs in cases where, due to machinery or spills, the enzyme product is subject to disruptive processes, including shear, impact or compression stresses as described by Meesters in Agglomeration of Enzymes, Micro-organisms and Flavours, Handbook of Power Technology, Volume 11 Granulation, edited by A.D. Salman et al., 2007.

Traditionally, biological actives (such as enzymes) have been formulated as either liquids or solids. Liquid formulations have the inherent advantage of suppressing enzyme dust formation, while for other properties, such as stability of the enzyme active, dry solid compositions are usually far superior because a biological active (such as an enzyme) can very effectively be separated from other ingredients, and dry compositions do not usually provide a medium in which the active is degraded. Although several solid formulations known in the art effectively limit the formation of active dust, it is a fact that active dust may still be released from the particles, e.g., as measured by the well-known Heubach Method, and this release increases when the solid formulation is broken apart as a result of disruptive stress during processing.

In this invention we combine the advantages of both liquids and solids with the result of lower active dust exposure, even after subjecting the particle containing the biological active to disruptive stress.

WO 2004/058933 describes making mechanical robust granules by impregnating (absorbing) a plasticizer onto a coating/surface containing a plasticizable polymer. The method described in the patent requires a complex and difficult additional step to the granulation process, and will only assure that the surface or coating of the granule is plasticized. Such plasticized coating/surface may reduce the tendency of the granule to release dust under physical stress/impacts as long as the granulate coating/surface is kept intact; however, it will not reduce dust emission if the granulate coating/surface is broken/cut, e.g., by a closing valve or by some milling process, both processes generally used, e.g., in the production of detergent powders.

WO 02/28991 describes particles comprising an active comprised in a visco-elastic liquid matrix, wherein the visco-elastic parameters η' (elastic parameter) and η" (viscous parameter) are between 10³ to 10¹⁴ Pa measured in a cone-and-plate rheometer at 25°C and a sinusoidal frequency, ω, of 1 Hz. Processing of such materials is inherently more difficult than materials not having visco-elastic properties, which implies that commonly used granulation methods like high-shear granulation, as described in US 4,106,991 and US 4,661,452 and fluid-bed processes, as described in US 5,324,649, cannot be used to make particles in the preferred range from 200 to 2000 µm. The visco-elastic properties will prevent a high-shear granulation process from working because a visco-elastic liquid matrix will not break upon impact, but merely deform with the consequence that essentially no particles in the desired size range is obtained. In a fluid-bed process the visco-elastic properties induces severe agglomeration, which also will lead to very low yields of particles having the desired size.

JP 2003000234 discloses enzyme particles prepared by a dry granulation method, comprising 0.05 to 5% w/w of propylene glycol; preferably 0.1 to 2% w/w of propylene glycol.

US 2007/111920 discloses a method for production of solid granulates with improved storage stability and abrasion resistance, based on the addition of hygroscopic polyols.

US 4,106,991 discloses preparation of enzyme granules using cellulose fibers to improve the granulation process and make a physically strong granule.

### SUMMARY OF THE INVENTION

The present invention provides, in a first aspect, a method for preparing a plurality of granules by mixer-granulation of a composition, wherein the composition comprises a biological active and 6-30% w/w of a non-volatile liquid having a vapor pressure of less than 1 kPa at 25°C, a melting point of 25°C or lower, and an elastic parameter, η', lower than 0.1 kPa, when measured in a cone-and-plate rheometer using a sinusoidal frequency, ω, of 1 Hz at 25°C; wherein mixer-granulation comprises progressive agglomeration of fine particles into larger granules; and wherein the biological active is an enzyme, a bacterial spore, a dehydrated yeast cell, or a dehydrated bacterial cell.

In an embodiment, the method further comprises applying a coating to the granules.

In another aspect, the invention also provides a plurality of granules obtainable by the method of the invention.

The plurality of granules exhibit reduced dust release upon exposure to mechanical stress, and are useful as ingredients in (powder) detergents.

Other aspects and embodiments of the invention are apparent from the description and examples.

### DETAILED DESCRIPTION

We have found that it is possible to prepare solid granules/particles containing a certain amount of non-volatile liquid and a biological active, such as an enzyme, where the release of active dust (of the biological active) after subjecting the particle to shear stress is comparable to that of an undisturbed particle. The active dust release before and after applying a shear stress is quantified by means of an Active Dust Analysis, as described in the Examples section. The analysis adds a pre-analysis step wherein the particles are compressed or even crushed and the coating is disrupted, providing a more well-defined picture of particle robustness against shear stress.

The reduced release of active dust after applying shear stress is surprising since methods known in the art only relate to reduced release of active dust from undisturbed, typically coated, particles containing biological actives.

Accordingly, one object of the present invention is to provide new and safer ways to use and handle biological actives selected from enzymes, bacterial spores, dehydrated yeast cells, and dehydrated bacterial cells. It is generally desired to separate biological actives from their surroundings until the moment when they are to be used in an application. This has been achieved by incorporating the active in discrete particles. Incorporation of the active in a particle also serves the purpose of lowering the amount of potentially harmful biologically active dust, which may be generated from the biological active. The present invention relates to such improved particles.

The particle/granule of the invention is an active dispersed in a wet solid matrix, wherein the solid matrix is wetted with a non-volatile liquid, such as glycerol. Such wetted, but still solid, particles exhibit lower release of active dust according to the "test method" (see the Examples) compared to a non-wetted reference particle.

In the present invention, the non-volatile liquid does not have the role of plasticizing (reducing the glass transition temperature) other solid ingredients of the formulation. Instead, it maintains the solid matrix wet so that it will deform plastically. US 4,106,991 explored the possibility of using a waxy component with a melting point above 30°C in order to obtain, to some extent, this plastic behavior. In this invention we use a non-volatile liquid to make wet solid particles that will be strong and plastic enough to sustain disruptive stress.

The present invention includes a mixer-granulation process, such as high-shear granulation, to achieve the final granulated product exhibiting low-dust properties.

### Definitions

The term "liquid" as used in the context of the invention is to be understood as a property of a material. A liquid material is defined as a material for which a certain amount of *stress, i.e.* force pr. unit area is obtained, when a deformation force, i.e. a strain is applied to the liquid material, as long as deformation occurs. As soon as deformation stops the stress level decreases immediately to the steady state level, which always will be *exactly zero.* Liquids are incapable of sustaining or maintaining an internal permanent stress in the liquid. The properties of a liquid are understood as properties of the liquid itself as applied to the granulation of coating (and not the properties of the resulting particle/coating).

The term "visco-elastic" as used in the context of this invention is to be understood as a property of the liquid. A liquid (fluid) is visco-elastic, when the time span for the stress in the material to reach exactly zero after a deformation is sufficiently large. Visco-elastic liquids may be described using a simple model containing two parameters η'(ω) and η"(w), which may easily be measured in a cone-and-plate rheometer (e.g., Bohlin Rheometer) for different sinusoidal frequencies ω. η'(ω) may be interpreted as the *elasticity parameter* of the visco-elastic fluid. This definition is acknowledged in the art, e.g., in Bird R.B., Armstrong R.C., Hassager O. "Dynamics of polymeric liquids", Volume 1: Fluid mechanics, John Wiley and Sons, Chapter 6, especially example 6.1.2.1, p 281, 1977; and used in WO 02/28991.

The term "viscosity" as used in the context of this invention is to be understood as a property of the liquid. The viscosity µ is given as the dynamic viscosity (e.g., in Pascal-second, Pa·s) measured in a cone-and-plate rheometer, e.g., Bohlin rheometer, using a shear rate of 1 s⁻¹ at 25°C unless otherwise stated. For Newtonian (or close to Newtonian) liquids the viscosity can be measured at other shear rates.

Surface tension, denoted as γ, is defined as the energy required to increase the surface area of a liquid per unit area and is commonly measured in mN/m (Kirk-Othmer Encyclopedia of Chemical Technology, "Surfactants"). The surface tension of a liquid in air can be measured by a person skilled in the art, and will be denoted γ_{LV}.

The wettability of a solid by a liquid is determined by the contact angle (θ) between the solid and the liquid. The work of adhesion is the work required to separate a unit area of an interface, and as described by Iveson et al. (2001), for θ >0°, γ_{SV}- γ_{SL}= γ_{LV} cos θ, where S, L and V refer to the solid, liquid and vapour phase, respectively. This term affects various granulation rate processes and granulate properties as known to the art (Iveson et al., 2001).

Materials that may be deformed extensively without breaking into small fragments, which potentially release airborne enzyme particles, have to be non-crystalline and additionally in a so called rubbery state. The transition between the arrested ("frozen") glass state and the rubbery state is called the *glass transition.* It starts to occur at a characteristic temperature called the glass transitions temperature Tg. The relation between the stickiness of a product, temperature and Tg has been extensively studied. At a temperature above Tg the product is in the rubbery state and has the desired breakage properties, but it is also sticky which prevents the material from being processed in industrial relevant processes such as spray dryers, fluid beds and extrusion processes and be transformed into a final product, which would cake together and not be fit for the final use.

The term "plurality of particles" as used in the context of the invention is the number of particles necessary to determine the particle size distribution with a reasonable accuracy, but at least 50 particles (randomly sampled), such as at least 100, 500 or 1000 particles. Typically, a plurality of particles is one or a few grams of particles. Particle size distribution may be measured using laser diffraction methods or optical digital imaging methods or sieve analysis. Unless otherwise indicated, the particle size is the volume based particle diameter (and the average particle size is the volume average particle diameter), which is the same as the weight based particle diameter if the densities of the particles are the same.

Unless otherwise indicated, or if it is apparent from the context that something else is meant, all percentages are percentage by weight (% w/w).

### Mixer-granulation process

The plurality of granules of the invention is prepared by a mixer-granulation process, which involves progressive agglomeration of fine particles into larger granules. A mixer-granulation process according to the invention is a process of growth/tumble agglomeration, wet granulation (not to be confused with extrusion), spray coating/layering, or powdering layering, as described in Handbook of Powder Technology, Volume 11, Granulation, 2007, ed. by A.D. Salman et. al., Chapter 9, Granulation Equipment by M. Jacob*.* Devices for such mixer-granulation processes are characterized by two distinct features:
a) As opposed to fluidized-bed equipment where a gas is used to create fluidization, mixing is carried out by mechanical means, sometimes with the use of agitators, and in particular cases the material is said to be mechanically fluidized.
b) The particles or individual granules (sometimes also referred to as agglomerates, grains or pellets), are formed directly *in* the mixing device. This is opposed to extrusion, where individual spherical or cylindrical pellets are only formed *after* a wet powder mass is forced to pass/flow through a single die, a series of dies, or a screen featuring many holes. The material flowing out of the die can break into individual particles spontaneously or a cutting device can be used. An optional feature of extrusion devices is that the cylindrical particles can be rounded (made spherical) by using a rotating friction plate or spheronizer/marumeriser (Handbook of Powder Technology, Volume 11, Granulation, 2007, ed. by A.D. Salman et. al., Chapters, Extrusion-Spheronisation by I. Wilson and S.L. Rough*).*

A particular case of mixer-granulation process is high-shear granulation. Mixer-granulation is a process for preparing granules (such as enzyme granules), which is well-known to the skilled person, and it is thoroughly described in various textbooks, such as in Handbook of Powder Technology; Particle size enlargement by C. E. Capes; Volume 1; 1980; Elsevier.

A mixer-granulation process includes adding a liquid, or a mixture of liquids, to a dry powder composition of, e.g., conventional granulating components, the biological active (e.g., an enzyme) being introduced either via the liquid or the powder or both. The liquid and the powder are mixed and as the moisture of the liquid is absorbed in the dry powder (the liquid acts as a binder), the components of the dry powder will start to adhere and agglomerate and particles will build up, forming granulates comprising the biological active. Such a process is described in US 4,106,991 and related documents EP 170360, EP 304332, EP 304331, WO 90/09440 and WO 90/09428. In a particular product of this process, wherein various high-shear mixers can be used as granulators, granulates consisting of enzyme, fillers and binders etc. are mixed with cellulose fibres to reinforce the particles to give the so-called T-granulate. Reinforced particles, being more robust, release less enzymatic dust.

An extrusion process has several disadvantages compared to mixer-granulation. The formulation flexibility is limited by the requirements of it being able to make a dough/paste with the right plastic properties to be extruded. Further the resulting particles are not very spherical unless further processing like e.g. spheronization is done, which also requires that the particles have the right plastic properties. For these reasons, the use of plasticizers, lubricants or moisturizing agents, including among others non-volatile liquids such as polyols, has been common for extrusion/spheronization. For enzyme particles, extrusion in practice results in large particles with a mean size of more than 750 µm, as using smaller holes in the die requires very high pressures and result in temperatures that deactivate the enzyme (see also Michael S. Showell (editor); Powdered detergents; Surfactant Science Series; 1998; vol. 71; page 140-142; Marcel Dekker).

In contrast, non-volatile liquids/plasticizers/moisturizing agents have not been used for mixer-granulation in significant amounts for a number of reasons, including a) plastic properties are not needed as the material does not need to flow through a die, and close to spherical particles are obtained directly from the mixer-granulation process, b) plastic properties are usually linked to sticky behavior, making the product difficult or impossible to process in the mixer-granulation or downstream operations (drying, transportation, etc.), c) too high plastic properties can result in too deformable, soft or weak granules that result in too high deformation, agglomeration, collapsing, etc.

We have surprisingly found that the addition of a non-volatile liquid in the mixer-granulation process in significantly high amounts can lubricate inter-particle forces in the granule while acting as binder, providing the agglomerate with plastic behavior (as opposed to brittle behavior), without necessarily plasticizing the internal components (decreasing their glass transition temperature). These properties result in a granule that releases less active dust upon receiving mechanical stress.

The non-volatile liquid can be added to the granulation liquid(s) (as a part of the granulation liquid) before applying the granulation liquid, or it can be added separately to the granulation process. The non-volatile liquid can be added directly into the mixer either before, after or simultaneously with the powder and other granulation liquids.

### Non-volatile Liquid

The non-volatile liquid, which is mixed with the biological active in the mixer-granulation process according to the invention, is a non-volatile liquid chemical compound or a mixture of non-volatile liquid chemical compounds, each having the properties as specified below.

The non-volatile liquid has a vapor pressure of less than 1 kPa at 25°C, and has an elastic parameter, η', lower than 0.1 kPa, when measured in a cone-and-plate rheometer (e.g., Bohlin Rheometer) using a sinusoidal frequency, ω, of 1 Hz at 25°C.

In a preferred embodiment, the vapor pressure of the non-volatile liquid is lower than 0.5 kPa at 25°C. In another preferred embodiment, the elastic parameter η' is lower than 10 Pa; e.g., lower than 1 Pa.

A low vapor pressure is preferred in order to slow down, as much as possible, the loss of liquid from the biological active containing particle from the moment it is produced until it is used. A low elastic parameter η' is preferred in order to allow processes such as breakage upon impact during high-shear granulation, thereby facilitating manufacturing.

The liquid has a melting point of 25°C (for components with a melting range this mean at least 50% of the component is in a liquid state) or below, more preferably below 20°C, even more preferably below 10°C, and most preferably below 5°C. The liquid is advantageously chosen such that it is in a liquid state at the conditions of use of the granule.

It is preferred that the non-volatile liquid is water soluble. The biological active component is typically added to the granulation as a solution or dispersion in water, and/or water is used as granulation aid in the granulation process. The non-volatile component can thus conveniently be added to the matrix by dissolving it in these aqueous liquids. The solubility of the non-volatile liquid in water should be at least 1% by weight at 25°C (i.e., 1 g dissolves in 99 g water), more preferred at least 10%, even more preferred at least 25% and most preferred at least 50%.

In an embodiment, the non-volatile liquid has a surface tension of at least 30 mN/m at 20°C (or at the melting point for liquid with higher melting point). Preferably, the surface tension is at least 40 mN/m; and more preferably at least 50 mN/m. A high surface tension is advantageous as it improves the binding effect of the liquid. This is important to balance the plastic behavior of the wet solid matrix with sufficient yield strength, thereby preventing disintegration of the granule under shear stress and decreasing active dust release.

In an embodiment, the non-volatile liquid has a dynamic viscosity of at least 0.001 Pa.s, more preferred at least 0.01 Pa s, and most preferred at least 0.1 Pa·s; measured at 25°C in a cone-and-plate rheometer at a shear rate of 1 s⁻¹. A high viscosity entails stronger binding effect of the liquid, providing improved strength of the particle resulting in reduced release of active dust.

In an embodiment, the non-volatile liquid is capable of wetting a mixture of the other components of the matrix. It is advantageous that the non-volatile liquid can wet a mixture of the other components in which it is incorporated, such that the liquid will spread and distribute easily in the matrix. This mean that a droplet of the liquid placed on a perfect surface of a mixture of the other components in the matrix should give a contact angle of less than 180 degrees, more preferred less than 135 degrees and most preferred less than 90 degrees.

In a preferred embodiment, the non-volatile liquids used according to the invention are polyols (polyhydric alcohols), for example alcohols with many hydroxyl groups such as glycerol, ethylene glycol, diethylene glycol, triethylene glycol, propylene glycol, dipropylene glycol, tripropylene glycol, polyethylene glycol, and polar low molecular weight organic compounds.

Most preferred are glycerol, triethylene glycol, propylene glycol, and polyethylene glycol (PEG) having an average molecular weight at or below about 1000.

As mentioned above, the non-volatile liquid may be a mixture of two or more compounds/liquids, each of which exhibit the properties of the non-volatile liquid of the invention.

The non-volatile liquids used in the present invention may be materials containing no or at least very little water. Water may be bound to the components of the liquid or it may contain water absorbed from a humid environment. The amount of water in the liquid will therefore depend on the components of the liquid, the hygroscopicity of the components and the humidity of the surrounding environment. Typically water is used as a processing aid, e.g., to moisture powder to be able to make granules by high shear mixing or carrying the active in water droplet to the surface of particles in fluid bed coating products. Most of this water is typically removed during processing/drying.

### Biological active

In the context of the present invention, biological actives are enzymes, and microorganisms selected from bacterial spores, dehydrated yeast cells, and dehydrated bacterial cells.

### Enzymes

The biological active may be one or more enzymes such as a protease, lipase, cutinase, an amylase, carbohydrase, cellulase, pectinase, mannanase, arabinase, galactanase, xylanase, DNase, perhydrolase, oxidase, e.g., a laccase, and/or peroxidase.

The enzyme may be a naturally occurring enzyme of bacterial or fungal origin, or it may be a variant derived from one or more naturally occurring enzymes by gene shuffling and/or by substituting, deleting or inserting one or more amino acids. Chemically modified or protein engineered mutants are included.

Preferably, the granule contains at least one enzyme in an amount of more than 0.5% w/w and less than 50% w/w active enzyme protein; more preferably in an amount of more than 0.6% w/w and less than 40% w/w active enzyme protein; more preferably in an amount of more than 0.75% w/w and less than 30% w/w active enzyme protein; and most preferably in an amount of more than 1% w/w and less than 25% w/w active enzyme protein.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme^{™}, Carezyme^{™}, and Celluclean^{™} (Novozymes A/S), Clazinase^{™}, and Puradax HA^{™} (Genencor International Inc.), and KAC-500(B)^{™} (Kao Corporation).

Proteases: Suitable proteases include those of bacterial, fungal, plant, viral or animal origin, e.g., vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from, e.g., family M4 or other metalloprotease, such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, *i.e.,* the Subtilisin family, the Thermitase family, the Proteinase K family, the Lantibiotic peptidase family, the Kexin family and the Pyrolysin family.

Examples of subtilases are those derived from *Bacillus* such as *Bacillus lentus, B. alkalophilus, B. subtilis, B. amyloliquefaciens, Bacillus pumilus* and *Bacillus gibsonii* described in; US7262042 and WO09/021867, and subtilisin lentus, subtilisin Novo, subtilisin Carlsberg, Bacillus licheniformis, subtilisin BPN', subtilisin 309, subtilisin 147 and subtilisin 168 described in WO89/06279 and protease PD138 described in WO93/18140. Other useful proteases may be those described in WO92/175177, WO01/016285, WO02/026024 and WO02/016547. Examples of trypsin-like proteases are trypsin (e.g., of porcine or bovine origin) and the *Fusarium* protease described in WO89/06270, WO94/25583 and WO05/040372, and the chymotrypsin proteases derived from *Cellumonas* described in WO05/052161 and WO05/052146.

A further preferred protease is the alkaline protease from *Bacillus lentus* DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Genencor Int.) such as those derived from *Bacillus amyloliquefaciens.*

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering).

Suitable commercially available protease enzymes include those sold under the trade names Alcalase^{®}, Duralase^{™}, Durazym^{™}, Relase^{®}, Relase^{®} Ultra, Savinase^{®}, Savinase^{®} Ultra, Primase^{®}, Polarzyme^{®}, Kannase^{®}, Liquanase^{®}, Liquanase^{®} Ultra, Ovozyme^{®}, Coronase^{®}, Coronase^{®} Ultra, Neutrase^{®}, Everlase^{®} and Esperase^{®} (Novozymes A/S), those sold under the tradename Maxatase^{®}, Maxacal^{®}, Maxapem^{®}, Purafect^{®}, Purafect Prime^{®}, Preferenz^{™}, Purafect MA^{®}, Purafect Ox^{®}, Purafect OxP^{®}, Puramax^{®}, Properase^{®}, Effectenz^{™}, FN2^{®}, FN3^{®}, FN4^{®}, Excellase^{®}, Opticlean^{®}, Optimase^{®}, and Excellenz P1000 (Danisco/DuPont), Axapem^{™} (Gist-Brocases N.V.), BLAP (sequence shown in Figure 29 of US5352604) and variants hereof (Henkel AG) and KAP *(Bacillus alkalophilus* subtilisin) from Kao.

Lipases and Cutinases: Suitable lipases and cutinases include those of bacterial or fungal origin. Chemically modified or protein engineered mutant enzymes are included. Examples include lipase from *Thermomyces, e.g.,* from *T. lanuginosus* (previously named *Humicola lanuginosa)* as described in EP258068 and EP305216, cutinase from *Humicola, e.g., H. insolens* (WO96/13580), lipase from strains of *Pseudomonas* (some of these now renamed to *Burkholderia), e.g., P. alcaligenes* or P. *pseudoalcaligenes* (EP218272), *P. cepacia* (EP331376), *P. sp.* strain SD705 (WO95/06720 & WO96/27002), *P. wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and S. *pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include include Lipolase^{™}, Lipex^{™}; Lipolex^{™} and Lipoclean^{™} (Novozymes A/S), Lumafast (originally from Genencor) and Lipomax (originally from Gist-Brocades).

Still other examples are lipases sometimes referred to as acyltransferases or perhydrolases, *e.g.,* acyltransferases with homology to *Candida antarctica* lipase A (WO10/111143), acyltransferase from *Mycobacterium smegmatis* (WO05/56782), perhydrolases from the CE 7 family (WO09/67279), and variants of the *M. smegmatis* perhydrolase in particular the S54V variant used in the commercial product Gentle Power Bleach from Huntsman Textile Effects Pte Ltd (WO10/100028).

Amylases: Suitable amylases are alpha-amylases or glucoamylases and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 3 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193. Other amylases which are suitable are hybrid alpha-amylase comprising residues 1-33 of the alpha-amylase derived from *B*. *amyloliquefaciens* shown in SEQ ID NO: 6 of WO 2006/066594 and residues 36-483 of the *B*. *licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, I201, A209 and Q264. Most preferred variants of the hybrid alpha-amylase comprising residues 1-33 of the alpha-amylase derived from *B*. *amyloliquefaciens* shown in SEQ ID NO: 6 of WO 2006/066594 and residues 36-483 of SEQ ID NO: 4 are those having the substitutions:
M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476. More preferred variants are those having a deletion in positions 181 and 182 or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:
N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl^{™}, Termamyl^{™}, Fungamyl^{™}, Stainzyme^{™}, Stainzyme Plus^{™}, Natalase^{™}, Liquozyme X and BAN^{™} (from Novozymes A/S), and Rapidase^{™} , Purastar^{™}/Effectenz^{™}, Powerase and Preferenz S100 (from Genencor International Inc./DuPont).

Lyase: The lyase may be a pectate lyase of bacterial or fungal origin. Chemically or genetically modified mutants are included. In a preferred embodiment the pectate lyase is derived from *Bacillus,* particularly *Bacillus substilis*, *B. licherniformis* or *B*. *agaradhaerens*, or a variant derived of any of these, e.g. as described in US 6,124,127, WO 1999/027083, WO 1999/027084, WO 2002/006442, WO 2002/092741, WO 2003/095638, Commercially available pectate lyases include XPect; Pectawash and Pectaway (Novozymes A/S).

Mannanase: Suitable mannanases include those of bacterial or fungal origin. Chemically or genetically modified mutants are included. The mannanase may be an alkaline mannanase of Family 5 or 26. It may be a wild-type from *Bacillus* or *Humicola,* particularly *B*. *agaradhaerens, B. licheniformis, B. halodurans, B. clausii,* or *H. insolens.* Suitable mannanases are described in WO 1999/064619. A commercially available mannanase is Mannaway (Novozymes A/S).

Deoxyribonuclease (DNase): Suitable deoxyribonucleases (DNases) are any enzyme that catalyzes the hydrolytic cleavage of phosphodiester linkages in the DNA backbone, thus degrading DNA. According to the invention, a DNase which is obtainable from a bacterium is preferred; in particular a DNase which is obtainable from a *Bacillus* is preferred; in particular a DNase which is obtainable from *Bacillus subtilis* or *Bacillus licheniformis* is preferred. Examples of such DNases are described in patent application WO 2011/098579 or in PCT/EP2013/075922.

Perhydrolases: Suitable perhydrolases are capable of catalyzing a perhydrolysis reaction that results in the production of a peracid from a carboxylic acid ester (acyl) substrate in the presence of a source of peroxygen (e.g., hydrogen peroxide). While many enzymes perform this reaction at low levels, perhydrolases exhibit a high perhydrolysis:hydrolysis ratio, often greater than 1. Suitable perhydrolases may be of plant, bacterial or fungal origin. Chemically modified or protein engineered mutants are included.

Examples of useful perhydrolases include naturally occurring *Mycobacterium* perhydrolase enzymes, or variants thereof. An exemplary enzyme is derived from *Mycobacterium smegmatis.* Such enzyme, its enzymatic properties, its structure, and variants thereof, are described in WO 2005/056782, WO 2008/063400, US 2008/145353, and US2007167344.

Peroxidases/Oxidases: Suitable peroxidases are comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from *Coprinopsis*, *e.g.*, from C. *cinerea* (EP 179,486), and variants thereof as those described in WO 93/24618, WO 95/10602, and WO 98/15257.

The peroxidases also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase of the invention is a chloroperoxidase. Preferably, the haloperoxidase is a vanadium haloperoxidase, *i.e.,* a vanadate-containing haloperoxidase. In a preferred method of the present invention the vanadate-containing haloperoxidase is combined with a source of chloride ion.

Haloperoxidases have been isolated from many different fungi, in particular from the fungus group dematiaceous hyphomycetes, such as *Caldariomyces, e.g.,* C. *fumago, Alternaria, Curvularia, e.g.,* C. *verruculosa* and C. *inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In an preferred embodiment, the haloperoxidase is derivable from *Curvularia* sp., in particular *Curvularia verruculosa* or *Curvularia inaequalis,* such as C. *inaequalis* CBS 102.42 as described in WO 95/27046; or C. *verruculosa* CBS 147.63 or C. *verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

Suitable oxidases include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be derived from plants, bacteria or fungi (including filamentous fungi and yeasts).

Suitable examples from fungi include a laccase derivable from a strain of *Aspergillus, Neurospora, e.g., N. crassa, Podospora, Botrytis, Collybia, Fomes, Lentinus, Pleurotus, Trametes, e.g., T. villosa* and *T. versicolor, Rhizoctonia, e.g., R. solani, Coprinopsis, e.g.,* C. *cinerea, C. comatus, C. friesii,* and C. *plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.* A laccase derived from *Coprinopsis* or *Myceliophthora* is preferred; in particular a laccase derived from *Coprinopsis cinerea,* as disclosed in WO 97/08325; or from *Myceliophthora thermophila,* as disclosed in WO 95/33836.

### Microorganisms

The biological active may be one or more microorganisms selected from bacterial spores, dehydrated yeast cells, and dehydrated bacterial cells.

Preferably, the one or more bacterial spores are *Bacillus* endospores; even more preferably the one or more spores are endospores of *Bacillus subtilis, Bacillus licheniformis, Bacillus amyloliquefaciens,* and/or *Bacillus megaterium.*

### Granule

The granules of the invention are small particles containing a biological active. The granules may be (roughly) spherical.

The granules typically have a (weight/volume average) diameter of 20-3000 µm, particularly 50-2000 µm, 100-1500 µm or 250-1200 µm.

In a particularly preferred embodiment, the plurality of granules has a (weight/volume average) diameter of 200-700 µm.

The granules are composed of a core, and optionally one or more coatings (outer layers) surrounding the core.

In an embodiment, the granules do not include a surfactant, a detergent builder, and/or a bleaching agent.

### Core

The core is made by mixer-granulation of a composition which includes, as a substantially homogenous mixture, the biological active and the non-volatile liquid. More specifically, the biological active and the non-volatile liquid are not separated, compartmentalized or arranged in discrete layers.

The composition comprises 6-30% w/w of a non-volatile liquid. The amount of the non-volatile liquid is at least 6% w/w of the composition; more preferably at least 7% w/w of the composition; and most preferably at least 9% w/w of the composition.

The upper limit of the non-volatile liquid is the amount necessary to saturate/fill the space between the solid particles in the matrix. The matrix must retain an overall non-liquid physical structure. For example, the amount of the non-volatile liquid may be less than 30% w/w of the matrix; most preferably less than 25% w/w of the matrix; and in particular less than 20% w/w of the matrix.

The core may include other granulation material(s) such as binder (e.g., synthetic polymer, wax, fat, or carbohydrate) filler, fibre material (cellulose or synthetic fibres), stabilizing agent, solubilising agent, suspension agent, viscosity regulating agent, light spheres, plasticizer, salt, lubricant, and/or fragrance.

The core may also include a crystalline material or a mixture of crystalline materials. A crystalline material, as used in the context of the invention, is a material which does not exhibit a glass transition with glycerol (e.g., as 50:50% w/w mixture with glycerol and measured by DSC); thus the crystalline material is not plasticized by glycerol. Examples of crystalline materials are silicates, e.g., micas or clays like kaolin, smectite, bentonite and talc; and inorganic salts like alkali metal sulfates, carbonates, nitrates and halides; alkaline earth metal sulfates, carbonates, nitrates and halides; transition metal sulfates, carbonates, nitrates and halides; and ammonium sulfates, carbonates, nitrates and halides; e.g., Na₂SO₄, K₂SO₄, CaSO₄, MgSO₄, ZnSO₄, (NH₄)₂SO₄, Na₂CO₃, NaHCO₃, K₂CO₃, KHCO₃, CaCO₃, MgCO₃, ZnCO₃, (NH₄)₂CO₃, NaNO₃, KNO₃, Ca(NO₃)₂, Mg(NO₃)₂, Zn(NO₃)₂, NH₄NO₃, NaCl, KCI, CaCl₂, MgCl₂, ZnCl₂, and NH₄Cl; or crystals like citrates, e.g., sodium or potassium citrate. Included are also the hydrates thereof.

The total amount of crystalline material(s) in the core/granule may be at least 10% w/w of the solid part of the composition of the core/granule. In an embodiment, the total amount of crystalline material(s) in the core/granule may be at least 20% w/w, such as at least 30% w/w, at least 40% w/w, or at least 50% w/w of the solid part of the composition of the core/granule.

The core may comprise a salt of a multivalent cation, a reducing agent, an antioxidant, a peroxide decomposing catalyst and/or an acidic buffer component, typically as a homogenous blend.

The core may consist of an inert particle with the matrix applied onto the surface of the inert particle, e.g., via seeded mixer granulation.

The core particle may have an average diameter of 20-3000 µm, particularly 50-2000 µm, 100-1500 µm or 250-1200 µm.

### Coating

The core of the granule may optionally be surrounded by at least one coating, e.g., to improve the storage stability, to reduce dust formation during handling, or for coloring the granule. The optional coating(s) may include a salt coating, or other suitable coating materials, such as polyethylene glycol (PEG), methyl hydroxy-propyl cellulose (MHPC) and polyvinyl alcohol (PVA). Examples of enzyme granules with multiple coatings are shown in WO 93/07263 and WO 97/23606.

The coating may be applied in an amount of at least 0.1% by weight of the core, e.g., at least 0.5%, 1% or 5%. The amount may be at most 100%, 70%, 50%, 40% or 30%.

The coating is preferably at least 0.1 µm thick, particularly at least 0.5 µm, at least 1 µm or at least 5 µm. In a particular embodiment the thickness of the coating is below 100 µm. In a more particular embodiment the thickness of the coating is below 60 µm. In an even more particular embodiment the total thickness of the coating is below 40 µm.

The coating should encapsulate the core unit by forming a substantially continuous layer. A substantially continuous layer is to be understood as a coating having few or no holes, so that the core unit it is encapsulating/enclosing has few or none uncoated areas. The layer or coating should in particular be homogeneous in thickness.

The coating can further contain other materials as known in the art, e.g., fillers, antisticking agents, pigments, dyes, plasticizers and/or binders, such as titanium dioxide, kaolin, calcium carbonate or talc.

A salt coating may comprise at least 60% by weight w/w of a salt, e.g., at least 65%, at least 70%, at least 75%, at least 80%, at least 85%, at least 90%, at least 95% or at least 99% by weight w/w.

The salt may be added from a salt solution where the salt is completely dissolved or from a salt suspension wherein the fine particles is less than 50 µm, such as less than 10 µm or less than 5 µm.

The salt coating may comprise a single salt or a mixture of two or more salts. The salt may be water soluble, in particular having a solubility at least 0.1 grams in 100 g of water at 20°C, preferably at least 0.5 g per 100 g water, *e.g.,* at least 1 g per 100 g water, *e.g.,* at least 5 g per 100 g water.

The salt may be an inorganic salt, e.g., salts of sulfate, sulfite, phosphate, phosphonate, nitrate, chloride or carbonate or salts of simple organic acids (less than 10 carbon atoms, e.g., 6 or less carbon atoms) such as citrate, malonate or acetate. Examples of cations in these salts are alkali or earth alkali metal ions, the ammonium ion or metal ions of the first transition series, such as sodium, potassium, magnesium, calcium, zinc or aluminium. Examples of anions include chloride, bromide, iodide, sulfate, sulfite, bisulfite, thiosulfate, phosphate, monobasic phosphate, dibasic phosphate, hypophosphite, dihydrogen pyrophosphate, tetraborate, borate, carbonate, bicarbonate, metasilicate, citrate, malate, maleate, malonate, succinate, lactate, formate, acetate, butyrate, propionate, benzoate, tartrate, ascorbate or gluconate. In particular alkali- or earth alkali metal salts of sulfate, sulfite, phosphate, phosphonate, nitrate, chloride or carbonate or salts of simple organic acids such as citrate, malonate or acetate may be used.

The salt in the coating may have a constant relative humidity at 20°C (also referred to as 'humidity fixed point') above 60%, particularly above 70%, above 80% or above 85%, or it may be another hydrate form of such a salt (e.g., anhydrate). The salt coating may be as described in WO 00/01793 or WO 2006/034710.

Specific examples of suitable salts are NaCl (CH_{20°C}=76%), Na₂CO₃ (CH_{20°C}=92%), NaNO₃ (CH_{20°C}=73%), Na₂HPO₄ (CH_{20°C}=95%), Na₃PO₄ (CH_{25°C}=92%), NH₄Cl (CH_{20°C} = 79.5%), (NH₄)₂HPO₄ (CH_{20°C} = 93.0%), NH₄H₂PO₄ (CH_{20°C} = 93.1%), (NH₄)₂SO₄ (CH_{20°C}=81.1%), KCI (CH_{20°C}=85%), K₂HPO₄ (CH_{20°C}=92%), KH₂PO₄ (CH_{20°C}=96.5%), KNO₃ (CH_{20°C}=93.5%), Na₂SO₄ (CH_{20°C}=93%), K₂SO₄ (CH_{20°C}=98%), KHSO₄ (CH_{20°C}=86%), MgSO₄ (CH_{20°C}=90%), ZnSO₄ (CH_{20°C}=90%) and sodium citrate (CH_{25°C}=86%). Other examples include NaH₂PO₄, (NH₄)H₃PO₄, CuSO₄, Mg(NO₃)₂ and magnesium acetate.

The salt may be in anhydrous form, or it may be a hydrated salt, i.e. a crystalline salt hydrate with bound water(s) of crystallization, such as described in WO 99/32595. Specific examples include anhydrous sodium sulfate (Na₂SO₄), anhydrous magnesium sulfate (MgSO₄), magnesium sulfate heptahydrate (MgSO₄ · 7H₂O), zinc sulfate heptahydrate (ZnSO₄ · 7H₂O), sodium phosphate dibasic heptahydrate (Na₂HPO₄ · 7H₂O), magnesium nitrate hexahydrate (Mg(NO₃)₂(6H₂O)), sodium citrate dihydrate and magnesium acetate tetrahydrate.

Preferably the salt is applied as a solution of the salt, e.g., using a fluid bed.

### Detergent composition

The granule of the invention may be added to and thus become a component of a detergent composition. When used in a detergent composition, the biological active of the granule is preferably a (detergent) enzyme or a bacterial spore.

In a specific aspect, the present invention provides a detergent additive comprising a granule of the present invention, as described herein.

In one embodiment, the invention is directed to detergent compositions comprising a granule of the present invention in combination with the additional cleaning composition components of a detergent builder and a surfactant.

The choice of additional components is within the skill of the artisan and includes conventional ingredients, including the exemplary non-limiting components set forth below.

The choice of components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

In one embodiment of the present invention, an enzyme containing granule of the invention may be added to a detergent composition in an amount corresponding to 0.001-200 mg of enzyme protein, such as 0.005-100 mg of enzyme protein, preferably 0.01-50 mg of enzyme protein, more preferably 0.05-20 mg of enzyme protein, even more preferably 0.1-10 mg of enzyme protein per liter of wash liquor.

### Surfactants

The detergent composition comprises one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and includes any conventional surfactant(s) known in the art. Any surfactant known in the art for use in detergents may be utilized.

When included therein the detergent will usually contain from about 1% to about 40% by weight, such as from about 5% to about 30%, including from about 5% to about 15%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein the detergent will usually contain from about 0.1% to about 10% by weight of a cationic surfactant. Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

When included therein the detergent will usually contain from about 0.2% to about 40% by weight of a non-ionic surfactant, for example from about 0.5% to about 30%, in particular from about 1% to about 20%, from about 3% to about 10%, such as from about 3% to about 5%, or from about 8% to about 12%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or *N*-acyl N-alkyl derivatives of glucosamine (glucamides, GA, or fatty acid glucamide, FAGA), as well as products available under the trade names SPAN and TWEEN, and combinations thereof.

When included therein the detergent will usually contain from about 0.1% to about 20% by weight of a semipolar surfactant. Non-limiting examples of semipolar surfactants include amine oxides (AO) such as alkyldimethylamineoxide, *N*-(coco alkyl)-*N,N*-dimethylamine oxide and *N-*(tallow-alkyl)-*N,N*-bis(2-hydroxyethyl)amine oxide, fatty acid alkanolamides and ethoxylated fatty acid alkanolamides, and combinations thereof.

When included therein the detergent will usually contain from about 0.1% to about 10% by weight of a zwitterionic surfactant. Non-limiting examples of zwitterionic surfactants include betaine, alkyldimethylbetaine, sulfobetaine, and combinations thereof.

### Hydrotropes

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see for example review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-5% by weight, such as about 0.5 to about 5%, or about 3% to about 5%, of a hydrotrope. Any hydrotrope known in the art for use in detergents may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

### Builders and Co-Builders

The detergent composition may contain about 0-65% by weight, such as about 5% to about 50% of a detergent co-builder. In a dish wash detergent, the level of builder is typically 40-65%, particularly 50-65%. The builder and/or co-builder may particularly be a chelating agent that forms water-soluble complexes with calcium and magnesium ions. Any builder and/or co-builder known in the art for use in laundry detergents may be utilized. Non-limiting examples of builders include citrates, zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI), and combinations thereof.

The detergent composition may also contain 0-50% by weight, such as about 5% to about 30%, of a detergent co-builder, or a mixture thereof. The detergent composition may include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N,N*'-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), N-(2-hydroxyethyl)iminodiacetic acid (EDG), aspartic acid-N-monoacetic acid (ASMA), aspartic acid-*N,N*-diacetic acid (ASDA), aspartic acid-N-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), N-methyliminodiacetic acid (MIDA), α-alanine-*N*, N-diacetic acid (α-ALDA), serine*-N*, N-diacetic acid (SEDA), isoserine-*N*, N-diacetic acid (ISDA), phenylalanine-*N*, N-diacetic acid (PHDA), anthranilic acid-*N*, N-diacetic acid (ANDA), sulfanilic acid-*N*, *N*-diacetic acid (SLDA) , taurine-/V, *N*-diacetic acid (TUDA) and sulfomethyl-*N*, *N*-diacetic acid (SMDA), N-(2-hydroxyethyl)-ethylidenediamine-*N*, *N*', N'-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 2009/102854, US 5,977,053.

### Bleaching Systems

The detergent may contain 0-50% by weight of a bleaching system. Any bleaching system known in the art for use in laundry detergents may be utilized. Suitable bleaching system components include bleaching catalysts, photobleaches, bleach activators, sources of hydrogen peroxide such as sodium percarbonate and sodium perborates, preformed peracids and mixtures thereof. Suitable preformed peracids include, but are not limited to, peroxycarboxylic acids and salts, percarbonic acids and salts, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides. Suitable examples are tetracetylethylene diamine (TAED), sodium 4-[(3,5,5-trimethylhexanoyl)oxy]benzene sulfonate (ISONOBS), diperoxy dodecanoic acid, 4-(dodecanoyloxy)benzenesulfonate (LOBS), 4-(decanoyloxy)benzenesulfonate, 4-(decanoyloxy)benzoate (DOBS), 4-(nonanoyloxy)-benzenesulfonate (NOBS), and/or those disclosed in WO 98/17767. A particular family of bleach activators of interest was disclosed in EP624154 and particulary preferred in that family is acetyl triethyl citrate (ATC). ATC or a short chain triglyceride like triacetin has the advantage that it is environmental friendly as it eventually degrades into citric acid and alcohol. Furthermore acetyl triethyl citrate and triacetin has a good hydrolytical stability in the product upon storage and it is an efficient bleach activator. Finally ATC provides a good building capacity to the laundry additive. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae: 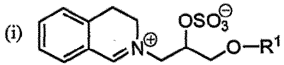 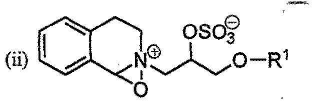 and mixtures thereof; wherein each R¹ is independently a branched alkyl group containing from 9 to 24 carbons or linear alkyl group containing from 11 to 24 carbons, preferably each R¹ is independently a branched alkyl group containing from 9 to 18 carbons or linear alkyl group containing from 11 to 18 carbons, more preferably each R¹ is independently selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl. Other exemplary bleaching systems are described, e.g., in WO 2007/087258, WO 2007/087244, WO 2007/087259 and WO 2007/087242. Suitable photobleaches may for example be sulfonated zinc phthalocyanine.

### Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide anti redeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575 and US 5,955,415. Salts of the above-mentioned polymers are also contemplated.

### Fabric hueing agents

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO 2005/03274, WO 2005/03275, WO 2005/03276 and EP 1876226. The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g., WO 2007/087257 and WO 2007/087243.

### Detergent enzyme(s)

The detergent additive as well as the detergent composition may comprise one or more (additional) enzymes, such as those mentioned above under the heading "Enzyme".

In general the properties of the selected enzyme(s) should be compatible with the selected detergent, (i.e., pH-optimum, compatibility with other enzymatic and non-enzymatic ingredients, etc.), and the enzyme(s) should be present in effective amounts.

The detergent enzyme(s) may be included in a detergent composition by adding separate additives containing one or more enzymes, or by adding a combined additive comprising all of these enzymes. A detergent additive of the invention, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

The detergent enzyme(s) may be included in a detergent composition by adding separate additives containing one or more enzymes, or by adding a combined additive comprising all of these enzymes. A detergent additive of the invention, *i.e.,* a separate additive or a combined additive is formulated as a granule of the invention.

### Adjunct materials

Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

Dispersants - The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

Dye Transfer inhibiting Agents - The detergent compositions of the present invention may also include one or more dye transfer inhibiting agents. Suitable polymeric dye transfer inhibiting agents include, but are not limited to, polyvinylpyrrolidone polymers, polyamine *N-*oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

Fluorescent whitening agent - The detergent compositions of the present invention will preferably also contain additional components that may tint articles being cleaned, such as fluorescent whitening agent or optical brighteners. Fluorescent whitening agents, also referred to as optical brighteners, optical brightening agents, or fluorescent brightening agents, are dyes that absorb light in the ultraviolet and violet region (usually 340-370 nm) of the electromagnetic spectrum, and re-emit light in the blue region (typically 420-470 nm). These agents are often used to enhance the appearance of color of fabric and paper, causing a whitening effect, making materials look less yellow by increasing the overall amount of blue light reflected.

Fluorescent whitening agents are well known in the art, and many such fluorescent agents are available commercially. Usually, fluorescent agents are supplied and used in the form of their alkali metal salts, for example, the sodium salts.

Preferred fluorescent agents are selected from the classes, distyrylbiphenyls, triazinylaminostilbenes, bis(1,2,3-triazol-2-yl)stilbenes, bis(benzo[b]furan-2-yl)biphenyls, 1,3-diphenyl-2-pyrazolines, thiophenediyl benzoxazole, and courmarins. The fluorescent agent is preferably sulfonated.

Preferred classes of fluorescent agents are: di-styryl biphenyl compounds, e.g., Tinopal^{™} CBS-X; di-amine stilbene di-sulphonic acid compounds, e.g., Tinopal DMS-X and Blankophor^{™} HRH; pyrazoline compounds, e.g., Blankophor SN; and thiophenediyl benzoxazole compounds, e.g., Tinopal OB.

Fluorescent agents are also described in McElhone, H.J. (2009), "Fluorescent Whitening Agents", Kirk-Othmer Encyclopedia of Chemical Technology, 1-16, DOI: 10.1002/0471238961.0612211513030512.a01.pub2.

Suitable fluorescent brightener levels include lower levels of from about 0.01, from 0.05, from about 0.1 or even from about 0.2 wt% to upper levels of 0.5 or even 0.75 wt%; such as from 0.01 wt% to 0.5 wt%.

Soil release polymers - The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore random graft co-polymers are suitable soil release polymers. Suitable graft co-polymers are described in more detail in WO 2007/138054, WO 2006/108856 and WO 2006/113314. Other soil release polymers are substituted polysaccharide structures especially substituted cellulosic structures such as modified cellulose deriviatives such as those described in EP 1867808 or WO 2003/040279. Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents - The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Other suitable adjunct materials include, but are not limited to, anti-shrink agents, anti-wrinkling agents, bactericides, binders, carriers, dyes, enzyme stabilizers, fabric softeners, fillers, foam regulators, perfumes, pigments, sod suppressors, solvents, and structurants for liquid detergents and/or structure elasticizing agents.

### Laundry soap bars

The granule of the invention may be added to laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, *i.e.,* if a solid object (e.g., laundry soap bar) is placed inside a container, the solid object does not change to fill the container it is placed in. The bar is a solid typically in bar form but can be in other solid shapes such as round or oval.

The laundry soap bar may contain one or more additional enzymes, protease inhibitors such as peptide aldehydes (or hydrosulfite adduct or hemiacetal adduct), boric acid, borate, borax and/or phenylboronic acid derivatives such as 4-formylphenylboronic acid, one or more soaps or synthetic surfactants, polyols such as glycerine, pH controlling compounds such as fatty acids, citric acid, acetic acid and/or formic acid, and/or a salt of a monovalent cation and an organic anion wherein the monovalent cation may be for example Na⁺, K⁺ or NH₄⁺ and the organic anion may be for example formate, acetate, citrate or lactate such that the salt of a monovalent cation and an organic anion may be, for example, sodium formate.

The laundry soap bar may also contain complexing agents like EDTA and HEDP, perfumes and/or different type of fillers, surfactants, e.g., anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as but not limited to: mixers, plodders, e.g., a two stage vacuum plodder, extruders, cutters, logo-stampers, cooling tunnels and wrappers. The invention is not limited to preparing the laundry soap bars by any single method. The premix of the invention may be added to the soap at different stages of the process. For example, the premix containing a soap, a granule of the invention, optionally one or more additional enzymes, a protease inhibitor, and a salt of a monovalent cation and an organic anion may be prepared and and the mixture is then plodded. The enzyme and optional additional enzymes may be added at the same time as the protease inhibitor for example in liquid form. Besides the mixing step and the plodding step, the process may further comprise the steps of milling, extruding, cutting, stamping, cooling and/or wrapping.

The present invention is further described by the following examples.

### EXAMPLES

Chemicals were commercial products of at least reagent grade.

### Test method

In order to evaluate whether the release of active dust increases after subjecting particles to mechanical forces, that would break or damage reference particles, a grinding method was applied. The test method uses a grinding device as a pre-analysis step before measuring active dust release, thereby providing a more drastic and realistic description of particle robustness against mechanical stress. The release of active dust is analyzed by the well-known Heubach method (as described by the Active Dust Analysis) before and after applying mechanical forces to an uncoated granulate by means of a grinding device. In this way the particle robustness is evaluated in the core itself, independently of the protective coating applied.

The grinding device is a MillMaster Grain Mill manufactured by Mashmaster Pty Ltd (Francis Hemeter, PO Box 1768, Coorparoo DC, Qld 4151, Australia) - some specifications of this instrument are:
- 130 mm precision machined rollers;
- 38 mm diameter Stainless Steel rollers; and
- 0.1 mm to 1.9 mm infinitely adjustable gap setting for precision control and accuracy.

The grinding device (MillMaster Grain Mill) has two dials which are eccentric adjustors for the desired gap. These eccentric adjustors have been modified in order to achieve gaps as low as 0 mm (from the originally available 0.1 mm to 1.9 mm). The gap is adjusted before performing a grinding assay by measuring it and ensuring that it is equal or smaller than half the D10, i.e., the 10% percentile of the particle size distribution (meaning that 10% of the volume of the particles has a size equal or less than the given value). For example, if the granules are sieved between 300-1200 µm, and the D10 is evaluated to be 400 µm, the gap must be adjusted below 200 µm. In the reported examples, the gap was adjusted to 150 µm in order to ensure the mentioned requirement with a safety margin, as the product to be analyzed was sieved between 300-1200 µm. In this way, the vast majority of particles will be shrinked while passing through the grinder, thereby suffering a high mechanical stress resulting in particle deformation and/or breakage.

The grinder device is used at a roller rotation speed of 30-40 rpm and the sample is fed at a rate of 4 to 6 g/min.

Other kinds of similar grinding devices may be used to grind the granules of the invention. What is important is that the reference granules (similar granule composition, but without the non-volatile liquid) is ground in the same way as the granules of the invention, in order to compare the dust levels.

### Sample preparation

The test method and the analysis of active dust is applied to a mixture of 10% w/w active-containing granules, and 90% placebo T-granules (meaning enzyme-free granules manufactured according to US 4,106,991 with the exception that sodium sulfate was used instead of sodium chloride), in order to simulate active-containing particles, possibly with plastic behavior, interacting with other particles of a different nature, as this will be the case in the application of the product.

The mixture is fed to the grinding device in sample size of 60 g. 50 g of the resulting grinded product are analyzed for active dust according to the Active Dust Analysis, resulting in the number "Active dust after grinding". Likewise, 50 g of undisturbed mixture (not-grinded active-containing particles) are analyzed by the Active Dust Analysis, resulting in the number "Active dust before grinding".

### Active Dust Analysis

The active dust release is analyzed by the well-known Heubach Type I dust meter by analyzing the activity of the biological active on the dust filter and converting the result into nanograms of biological active divided by grams of sample. In this way the result is independent of possible non-active dust generated by the placebo T-granule in the mixture.

The weighed out sample amount is placed in a rotating drum containing three integrated blades. A horizontal stream passes through the drum with airflow at 20 L/min. The airflow leads the finest particles further through a non-rotating, horizontal glass column in which the largest particles are separated. The airborne dust is led further and collected on a filter in the filter house. The amount of biological active dust on the filter is determined by means of an analytical method for dust filters for the biological active in question.

### Conditions of Analysis:

| | |
|---|---|
| Temperature: | Room temperature |
| Sample amount: | 50.0 g |
| Air flow: | 20 L/min. |
| Speed of rotation: | 30 rpm |
| Time of analysis: | 5 min. |
| Humidity of air: | 30-70 %RH |
| Fiber glass filter: | 5 cm GF92 |

### EXAMPLE 1 (Reference)

A reference T-granule was prepared according to US 4,106,991 (sodium sulfate was used instead of sodium chloride), containing only water as granulation liquid, and therefore does not meet the claimed composition. The protease (Savinase^{™}) content and active enzyme dust release before and after applying the "test method" are shown in Table 2. It is clear from the results that the lack of non-volatile liquid results in high release of active enzyme dust.

### EXAMPLE 2 (Reference)

A T-granule was prepared according to US 4,106,991 (sodium sulfate was used instead of sodium chloride), containing approximately 5.3% of glycerol in the granules (core) on dry basis (not accounting for possible water in the granules). The protease (Savinase^{™}) content and active dust release before and after applying the "test method" are shown in Table 2. As shown, this amount of the non-volatile liquid reduces the release of active enzyme dust.

### EXAMPLE 3

A T-granule was prepared according to US 4,106,991 (sodium sulfate was used instead of sodium chloride), containing approximately 6.3% of glycerol in the granules on dry basis (not accounting for possible water in the granules). The protease (Savinase^{™}) content and active dust release before and after applying the "test method" are shown in Table 2. As shown, this amount of the non-volatile liquid reduces the release of active enzyme dust.

### EXAMPLE 4

A T-granule was prepared according to US 4,106,991 (sodium sulfate was used instead of sodium chloride), containing approximately 11.8% of glycerol in the granules on dry basis (not accounting for possible water in the granules). The protease (Savinase^{™}) content and active dust release before and after applying the "test method" are shown in Table 2. As shown, this amount of the non-volatile liquid reduces the release of active enzyme dust.

### EXAMPLE 5

A T-granule was prepared according to US 4,106,991 (sodium sulfate was used instead of sodium chloride), containing approximately 12.1% of glycerol in the granules on dry basis (not accounting for possible water in the granules), and with a protease (Savinase^{™}) content as shown in Table 2. Active dust release before and after applying the "test method" are also shown in Table 2. As shown, this amount of the non-volatile liquid reduces the release of active enzyme dust.

### EXAMPLE 6

A T-granule was prepared according to US 4,106,991 (sodium sulfate was used instead of sodium chloride), containing approximately 13.0% of PEG400 in the granules on dry basis (not accounting for possible water in the granules). The protease (Savinase^{™}) content and active dust release before and after applying the "test method" are shown in Table 2. As shown, this amount of the non-volatile liquid reduces the release of active enzyme dust.

### EXAMPLE 7

A T-granule was prepared according to US 4,106,991 (sodium sulfate was used instead of sodium chloride), containing approximately 16.8% of PEG400 in the granulaes on dry basis (not accounting for possible water in the granules). The protease (Savinase^{™}) content and active dust release before and after applying the "test method" are shown in Table 2. As shown, this amount of the non-volatile liquid reduces the release of active enzyme dust.

### EXAMPLE 8

A T-granule was prepared according to US 4,106,991 (sodium sulfate was used instead of sodium chloride), containing approximately 11.8% of a 1:1 mixture of PEG600 and glycerol in the granules on dry basis (not accounting for possible water in the granules). The protease (Savinase^{™}) content and active dust release before and after applying the "test method" are shown in Table 2. As shown, this amount of the non-volatile liquid reduces the release of active enzyme dust.

### EXAMPLE 9

A T-granule was prepared according to US 4,106,991 (sodium sulfate was used instead of sodium chloride), containing approximately 11.8% of a 1:1 mixture of PEG1000 and glycerol in the granules on dry basis (not accounting for possible water in the granules). The protease (Savinase^{™}) content and active dust release before and after applying the "test method" are shown in Table 2. As shown, this amount of the non-volatile liquid reduces the release of active enzyme dust.

### EXAMPLE 10

A T-granule was prepared according to US 4,106,991 (sodium sulfate was used instead of sodium chloride), containing approximately 9% of MPG (propylene glycol) in the granules on dry basis (not accounting for possible water in the granules). The protease (Savinase^{™}) content and active dust release before and after applying the "test method" are shown in Table 2. As shown, this amount of the non-volatile liquid reduces the release of active enzyme dust.

### EXAMPLE 11

A T-granule was prepared according to US 4,106,991 (sodium sulfate was used instead of sodium chloride), containing approximately 11.8% of a 1:1 mixture of tripropylene glycol and glycerol in the granules on dry basis (not accounting for possible water in the granules). The protease (Savinase^{™}) content and active dust release before and after applying the "test method" are shown in Table 2. As shown, this amount of the non-volatile liquid reduces the release of active enzyme dust.

**Table 1. Properties of the non-volatile liquids used in Examples 1-11.**

| Non-volatile liquid | Vapor pressure at 25°C | Elastic parameter η' at 25°C and ω=1 Hz | Dynamic viscosity at 25°C and shear rate 1 s⁻¹ | Surface tension with air at 20°C |
|---|---|---|---|---|
| Glycerol | < 1 kPa | < 0.1 kPa | > 0.5 Pa·s | > 60 mN/m |
| PEG400 | < 1 kPa | < 0.1 kPa | > 0.1 Pa·s | > 40 mN/m |
| MPG | < 1 kPa | < 0.1 kPa | > 0.01 Pa·s | > 35 mN/m |

**Table 2. Release of active enzyme dust before/after applying the "test method" to the granules.**

| Example | Non-volatile liquid | Amount of non-volatile liquid (% w/w) | Protease content (µg/g) | Active dust before grinding (µg/g) | Active dust after grinding (µg/g) | Active dust fraction after grinding (ppm) |
|---|---|---|---|---|---|---|
| 1 (Ref.) | **None (reference)** | 0 | 74000 | 0.29 | 43.40 | **586** |
| 2 (Ref.) | Glycerol | 5.3 | 72000 | 0.25 | 13.90 | **193** |
| 3 | Glycerol | 6.3 | 65000 | 0.16 | 5.36 | **82** |
| 4 | Glycerol | 11.8 | 102000 | 0.11 | 1.16 | **11** |
| 5 | Glycerol | 12.1 | 73000 | 0.60 | 0.29 | **4** |
| 6 | PEG400 | 13.0 | 63000 | 0.29 | 2.15 | **34** |
| 7 | PEG400 | 16.8 | 56000 | 0.25 | 0.36 | **6** |
| 8 | PEG600 + Glycerol (1:1) | 11.8 | 82000 | 0.15 | 3.10 | **38** |
| 9 | PEG1000 + Glycerol (1:1) | 11.8 | 95000 | 0.19 | 3.99 | **42** |
| 10 | MPG | 9.0 | 75000 | 0.45 | 1.15 | **15** |
| 11 | TPG + Glycerol (1:1) | 11.8 | 77000 | 0.22 | 5.61 | **73** |

| | | | | | | |
|---|---|---|---|---|---|---|
| "Active dust fraction after grinding" is calculated as: "Active dust after grinding" / "Protease content" | | | | | | |

## Claims

1. A method for preparing a plurality of granules by mixer-granulation of a composition, wherein the composition comprises a biological active and 6-30% w/w of a non-volatile liquid having a vapor pressure of less than 1 kPa at 25°C, a melting point of 25°C or lower, and an elastic parameter, η', lower than 0.1 kPa, when measured in a cone-and-plate rheometer using a sinusoidal frequency, ω, of 1 Hz at 25°C;
wherein mixer-granulation comprises progressive agglomeration of fine particles into larger granules; and wherein the biological active is an enzyme, a bacterial spore, a dehydrated yeast cell, or a dehydrated bacterial cell.

2. The method of claim 1, wherein the enzyme is selected from the group consisting of a protease, lipase, cutinase, amylase, carbohydrase, cellulase, pectinase, mannanase, arabinase, galactanase, xylanase, DNase, perhydrolase, oxidase, laccase, peroxygenase, haloperoxidase, and peroxidase.

3. The method of claim 1 or 2, wherein the non-volatile liquid is a polyol.

4. The method of any of claims 1-3, wherein the non-volatile liquid is a polyol selected from the group consisting of glycerol, ethylene glycol, diethylene glycol, triethylene glycol, propylene glycol, dipropylene glycol, tripropylene glycol, and polyethylene glycol.

5. The method of any of claims 1-4, wherein the composition comprises 7-30% w/w of the non-volatile liquid.

6. The method of any of claims 1-5, wherein the composition comprises at least 10% w/w crystalline material relative to the non-liquid part of the composition, which is one or more inorganic salts or clays, such as one or more salts of sulfate, carbonate, nitrate, chloride; and/or kaolin, smectite, bentonite, talc.

7. The method of any of claims 1-6, which further comprises applying a coating to the granules, wherein the coating makes up 5-70% w/w relative to the granules and comprises at least 60% w/w of a salt having a constant relative humidity at 20°C of at least 60%.

8. The method of any of claims 1-7, wherein the volume average diameter of the plurality of granules is 200-700 µm.

9. The method of any of the preceding claims, wherein the non-volatile liquid has a surface tension of at least 30 mN/m at 20°C or at the melting point for liquids with a higher melting point.

10. The method of any of the preceding claims, wherein the non-volatile liquid has a dynamic viscosity of at least 0.001 Pa.s, when measured at 25°C in a cone-and-plate rheometer at a shear rate of 1 s⁻¹.

11. A plurality of granules obtainable by the method of any of the preceding claims.

12. A particulate detergent composition comprising a detergent builder, a surfactant, and a plurality of granules according to claim 11.

## Patentansprüche

1. Verfahren zum Herstellen einer Vielzahl von Körnchen durch Mischgranulieren einer Zusammensetzung, wobei die Zusammensetzung einen biologischen Wirkstoff und 6-30% Gew./Gew. einer nichtflüchtigen Flüssigkeit mit einem Dampfdruck von weniger als 1 kPa bei 25°C, einen Schmelzpunkt von 25°C oder weniger und einen elastischen Parameter η' von weniger als 0,1 kPa aufweist, wenn in einem Kegel/Platte-Rheometer unter Verwendung einer sinusförmigen Frequenz w von 1 Hz bei 25°C gemessen wird;
wobei das Mischgranulieren eine fortschreitende Agglomeration feiner Partikel zu größeren Körnchen umfasst; und wobei der biologische Wirkstoff ein Enzym, eine Bakterienspore, eine dehydratisierte Hefezelle oder eine dehydratisierte Bakterienzelle ist.

2. Verfahren nach Anspruch 1, wobei das Enzym aus der Gruppe ausgewählt ist, die aus einer Protease, Lipase, Cutinase, Amylase, Carbohydrase, Cellulase, Pectinase, Mannanase, Arabinase, Galactanase, Xylanase, DNase, Perhydrolase, Oxidase, Laccase, Peroxygenase, Haloperoxidase und Peroxidase besteht.

3. Verfahren nach Anspruch 1 oder 2, wobei die nichtflüchtige Flüssigkeit ein Polyol ist.

4. Verfahren nach einem beliebigen der Ansprüche 1-3, wobei die nichtflüchtige Flüssigkeit ein Polyol ist, das aus der Gruppe ausgewählt ist, die aus Glycerin, Ethylenglykol, Diethylenglykol, Triethylenglykol, Propylenglykol, Dipropylenglykol, Tripropylenglykol und Polyethylenglykol besteht.

5. Verfahren nach einem beliebigen der Ansprüche 1-4, wobei die Zusammensetzung 7-30% Gew./Gew. der nichtflüchtigen Flüssigkeit umfasst.

6. Verfahren nach einem beliebigen der Ansprüche 1-5, wobei die Zusammensetzung mindestens 10% Gew./Gew. kristallines Material relativ zum nichtflüssigen Teil der Zusammensetzung umfasst, welches ein oder mehrere anorganische Salze oder Tone, wie ein oder mehrere Salze von Sulfat, Carbonat, Nitrat, Chlorid, und/oder Kaolin, Smektit, Bentonit, Talkum ist.

7. Verfahren nach einem beliebigen der Ansprüche 1-6, das des Weiteren Aufbringen einer Beschichtung auf die Körnchen umfasst, wobei die Beschichtung 5-70% Gew./Gew. relativ zu den Körnchen ausmacht und mindestens 60% Gew./Gew. eines Salzes mit einer konstanten relativen Feuchtigkeit bei 20°C von mindestens 60% umfasst.

8. Verfahren nach einem beliebigen der Ansprüche 1-7, wobei der mittlere Volumendurchmesser der Vielzahl von Körnchen 200-700 µm beträgt.

9. Verfahren nach einem beliebigen der voranstehenden Ansprüche, wobei die nichtflüchtige Flüssigkeit eine Oberflächenspannung von mindestens 30 mN/m bei 20°C oder beim Schmelzpunkt für Flüssigkeiten mit einem höheren Schmelzpunkt aufweist.

10. Verfahren nach einem beliebigen der voranstehenden Ansprüche, wobei die nichtflüchtige Flüssigkeit eine dynamische Viskosität von mindestens 0,001 Pa·s aufweist, wenn bei 25°C in einem Kegel/Platte-Rheometer bei einer Schergeschwindigkeit von 1 s⁻¹ gemessen wird.

11. Vielzahl von Körnchen, erhältlich durch das Verfahren gemäß einem beliebigen der voranstehenden Ansprüche.

12. Teilchenförmige Detergenszusammensetzung, die einen Detergensgerüststoff, ein oberflächenaktives Mittel und eine Vielzahl von Körnchen gemäß Anspruch 11 umfasst.

## Revendications

1. Méthode pour préparer une pluralité de granules par granulation d'une composition dans un mélangeur, dans laquelle la composition comprend un principe actif biologique et 6 à 30 % en poids/poids d'un liquide non volatil ayant une pression de vapeur inférieure à 1 kPa à 25 °C, un point de fusion de 25 °C ou moins, et un paramètre élastique η' inférieur à 0,1 kPa, lors d'une mesure dans un rhéomètre à cône et plan utilisant une fréquence sinusoïdale ω de 1 Hz à 25 °C ;
dans laquelle la granulation dans un mélangeur comprend l'agglomération progressive de fines particules en granules plus gros ; et dans laquelle le principe actif biologique est une enzyme, un spore bactérien, une cellule de levure déshydratée, ou une cellule bactérienne déshydratée.

2. Méthode selon la revendication 1, dans laquelle l'enzyme est choisie dans le groupe constitué par une protéase, une lipase, une cutinase, une amylase, une carbohydrase, une cellulase, une pectinase, une mannanase, une arabinase, une galactanase, une xylanase, une DNase, une perhydrolase, une oxydase, une laccase, une peroxygénase, une haloperoxydase, et une peroxydase.

3. Méthode selon la revendication 1 ou 2, dans laquelle le liquide non volatil est un polyol.

4. Méthode selon l'une quelconque des revendications 1 à 3, dans laquelle le liquide non volatil est un polyol choisi dans le groupe constitué par le glycérol, l'éthylèneglycol, le diéthylèneglycol, le triéthylèneglycol, le propylèneglycol, le dipropylèneglycol, le tripropylèneglycol, et le polyéthylèneglycol.

5. Méthode selon l'une quelconque des revendications 1 à 4, dans laquelle la composition comprend 7 à 30 % en poids/poids du liquide non volatil.

6. Méthode selon l'une quelconque des revendications 1 à 5, dans laquelle la composition comprend au moins 10 % en poids/poids de matériau cristallin par rapport à la partie non liquide de la composition, qui est un ou plusieurs sels inorganiques ou argiles, tels qu'un ou plusieurs sels sulfates, carbonates, nitrates, chlorures ; et/ou le kaolin, la smectite, la bentonite, le talc.

7. Méthode selon l'une quelconque des revendications 1 à 6, qui comprend en outre l'application d'un revêtement aux granules, dans laquelle le revêtement constitue jusqu'à 5 à 70 % en poids/poids des granules et comprend au moins 60 % en poids/poids d'un sel ayant une humidité relative constante à 20 °C d'au moins 60 %.

8. Méthode selon l'une quelconque des revendications 1 à 7, dans laquelle le diamètre moyen en volume de la pluralité de granules est de 200 à 700 µm.

9. Méthode selon l'une quelconque des revendications précédentes, dans laquelle le liquide non volatil a une tension de surface d'au moins 30 mN/m à 20 °C ou au point de fusion pour les liquides ayant un point de fusion plus élevé.

10. Méthode selon l'une quelconque des revendications précédentes, dans laquelle le liquide non volatil a une viscosité dynamique d'au moins 0,001 Pa.s lors d'une mesure à 25 °C dans un rhéomètre à cône et plan à une vitesse de cisaillement de 1 s⁻¹.

11. Pluralité de granules pouvant être obtenus par la méthode de l'une quelconque des revendications précédentes.

12. Composition détergente particulaire comprenant un adjuvant pour détergent, un tensioactif, et une pluralité de granules selon la revendication 11.
